# EUROPEAN PATENT SPECIFICATION

(11) **EP 1 343 416 B1**
(45) Date of publication and mention of the grant of the patent: **15.10.2008**
(21) Application number: 01992360.6
(22) Date of filing: 21.12.2001
(51) Int. Cl.: A61F 2/06, A61M 25/09, A61M 25/00, A61M 27/00, A61F 2/00, A61B 19/00

(54) **STENT DELIVERY SYSTEM**
STENTABLEGESYSTEM
SYSTEME DE POSE DE STENT

(30) Priority: 22.12.2000 US 258196 P; 09.01.2001 US 260833 P
(43) Date of publication of application: 17.09.2003
(73) Proprietor: APPLIED MEDICAL RESOURCES CORPORATION, Rancho Santa Margarita, CA 92688 (US)
(72) Inventor: EWERS, Richard, C., Fullerton, CA 92833 (US)
(74) Representative: Fitchett, Stuart Paul
(86) International application number: PCT/US2001/050265
(87) International publication number: WO 2002/051302

(56) References cited:
- EP-A- 0 880 949
- EP-A1- 0 516 189
- EP-A2- 0 819 411
- WO-A-93/15872
- WO-A-94/06357
- WO-A-98/39055
- WO-A-99/47075
- US-A- 4 713 049
- US-A- 5 019 102
- US-A- 5 964 744
- US-A- 6 136 006

## Description

### BACKGROUND OF THE INVENTION

### 1. Field of the Invention

The present invention relates generally to medical and surgical devices, and more specifically to ureteral stents and the deployment thereof.

### 2. Description of Prior Art and Related Information

Stents are deployed in the ureter to provide fluid communication between the kidney and the bladder. A conventional ureteral stent usually consists of a cylindrical body defining a lumen therein. The conventional stent consists of a single pigtail anchor located at the distal end. A distal tip of the common stent includes one or more holes that are in fluid communication with the lumen.

The conventional ureteral stent is typically deployed by threading it over a guidewire that has been inserted through the urethra to reach the kidney. The stent is advanced distally by pushing it forward along the guidewire. In a common approach, the portion of the stent outside the urethra is manually advanced along the guidewire. Since stents are formed at a predetermined length, namely, to extend between the kidney and the bladder, deployment is problematic especially when there remains no further portion of the stent external to the urethra to be advanced. One solution has been to employ a rigid tube, or pusher, that is threaded over a guidewire proximally of the stent. The tube is advanced forward which then pushes a proximal end of the stent distally. A stent introducer of this kind is disclosed in US 4.713.049.

### SUMMARY OF THE INVENTION

The present invention provides structures which overcome the deficiencies of the prior art, according to the appended claims.

A surgical combination for stenting a ureter is provided. The combination comprises a guidewire having a distal end. The guidewire is adapted to be positioned in the ureter. The combination further comprises a stent and a positioner. The stent has a body, a distal anchor, and a radiopaque marker disposed around the body proximally of the distal anchor. The marker is sized and configured for distal movement along the guidewire to an operative site. The positioner has a tubular configuration and is adapted for distal movement along the guidewire to hold the marker at the operative site when the guidewire is wire is withdrawn from the ureter.

The distal anchor has a both straight configuration and a curled configuration. The distal anchor is disposed in the straight configuration when the marker is moved distally along the guidewire. The distal anchor is disposed in the curled configuration when the guidewire is withdrawn from the operative site. The distal anchor is preferably biased to the curled configuration.

In another aspect, a combination is provided which enables deployment of a stent without having to retract a guidewire. Thus, the guidewire may remain inserted while the stent is independently released and deployed. The combination includes a guidewire, a stent having a proximal end and a distal end, and an insertion tube including a first lumen and a second lumen. The first lumen is sized and configured to receive the guidewire. The second lumen of the tube is adapted to receive at least a portion of the stent in a releasably locked relationship. Portions of the second tube define a scythe or side port which extends into the second lumen. A rod is disposed in the second lumen and movable between a first locking position wherein the rod engages the portion of the stent within the second lumen, and a second releasing position wherein the rod disengages the portion of the second stent in the second lumen. The portion of the stent engaged by the rod comprises a distal end of the stent.

The invention, now having been briefly summarized, may be better appreciated by the following description of preferred embodiments and reference to the associated drawings.

### BRIEF DESCRIPTION OF THE DRAWINGS

FIG. 1 is a side elevation view of a first preferred embodiment of a mesh stent according to the present invention;
FIG. 2 is a side elevation view of a first preferred embodiment of a positioner for deploying the stent over a guidewire;
FIG. 3 is a side elevation view illustrating the first preferred stent in one preferred position being moved over a guidewire by the first preferred positioner;
FIG. 4 is a schematic view of a guidewire operatively positioned through a bladder, a ureter and into a kidney;
FIG. 5 is a side elevation view illustrating one method for moving the stent along the guide using the positioner;
FIG. 6 is a side elevation view illustrating a step of releasing the first preferred stent from the guidewire;
FIG. 7 is a side elevation view of the first preferred stent released from the guidewire and automatically forming a coil in the kidney;

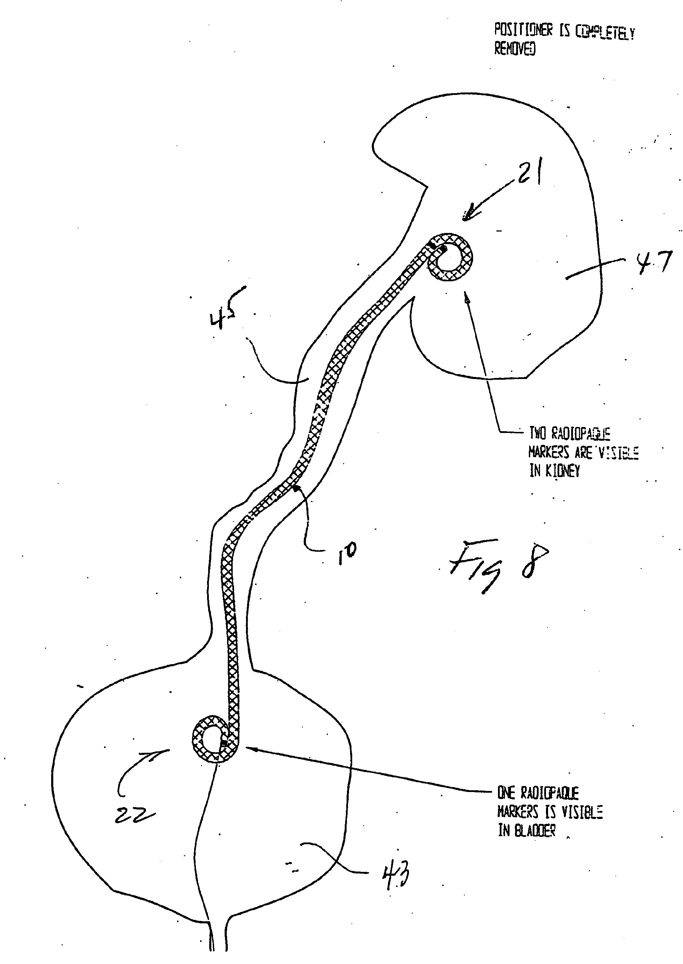

FIG. 8 is a side elevation view illustrating removal of the guidewire and first preferred positioner leaving the first preferred stent operatively disposed with anchor coils in both the kidney and the bladder;
FIG. 9A is a side elevation view of the first preferred stent;
FIG. 9B is a side elevation view of the first preferred positioner;
FIG. 9C is a side elevation view similar to Fig. 3 and illustrating an alternative method for positioning a portion of the stent on the guidewire;
FIG. 10A is a side elevation view of the first preferred stent;
FIG. 10B is a side elevation view of the first preferred positioner;
FIG. 10C is a side elevation view similar to Fig. 3 and Fig. 9 and illustrating a further method for moving the stent along the guidewire;
FIG. 11 is a side elevation view similar to Fig. 5 and illustrating the positioning of the first preferred stent in the kidney with the orientation illustrated in Fig. 10;
FIG. 12 is a side elevation view illustrating formation of a distal anchor coil prior to removal of the guidewire from the first preferred stent;
FIG. 13 is a side elevation view illustrating removal of the guidewire into the first preferred positioner to release the first preferred stent from the guidewire; and
FIG. 14 is a side elevation view of a second preferred embodiment of a stent;
FIG. 15 is an axial, cross-sectional view of a second preferred positioner;
FIG. 16 is a side elevation view of a guidewire;
FIG. 17 is a perspective view of the second preferred positioner;

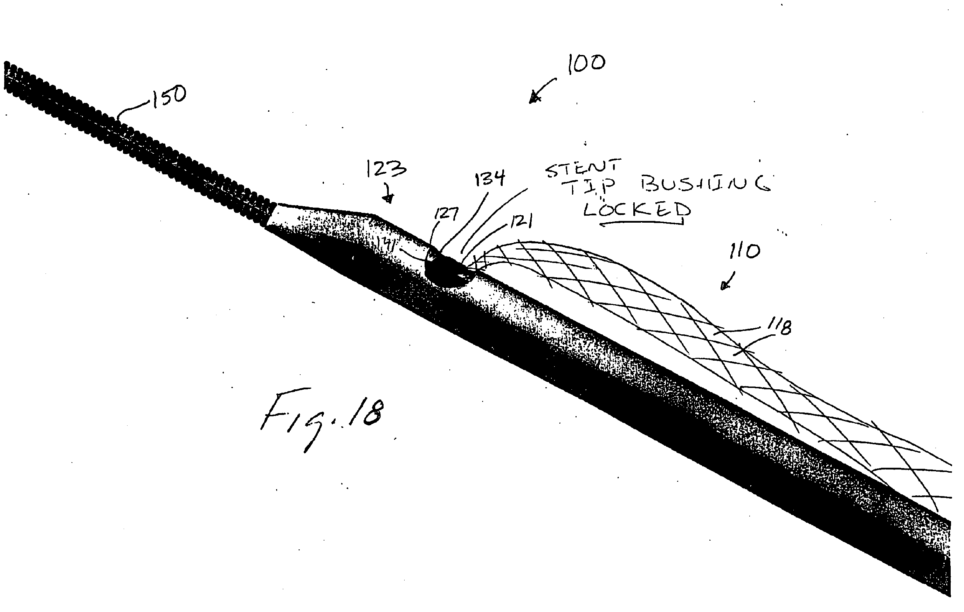

FIG. 18 is a perspective view of a second preferred embodiment of a stent deployment system; and
FIG. 19 is a perspective view of the second preferred embodiment of the stent deployment system with the stent released from the positioner and, thus, deployed.

### DETAILED DESCRIPTION OF PREFERRED EMBODIMENTS AND BEST MODE OF THE INVENTION

The invention can now be better understood with the following detailed description.

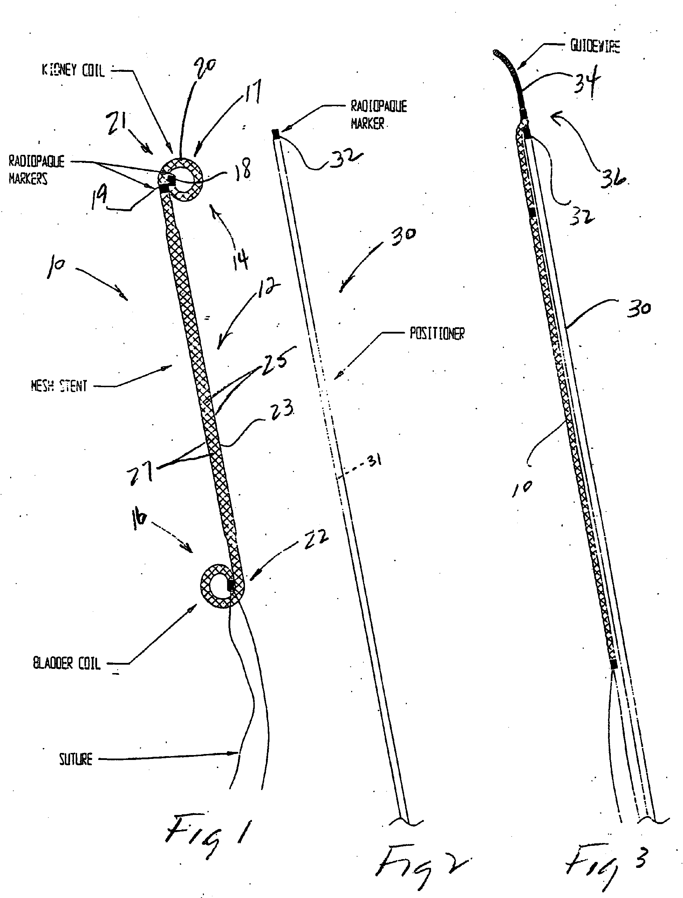

A ureteral stent of the invention is illustrated in Figure 1 and designated by the reference numeral 10. The stent 10 has an elongate configuration defined by a body 12 extending between a distal end 14 and proximal end 16. A distal section 17 and proximal section 22 of the stent 10 are of particular interest to the present invention. This section 17 includes radiopaque markers, or sleeves, 18 and 19, and a distal portion 20 of the body 12 which extends therebetween. The marker 18 is disposed at the distal end 14 and may function as a guide bushing. The marker 19 is disposed proximally of the marker 18 and may have a tubular configuration so that it can also function as a guide bushing. A distal anchor 21 and the proximal section, or proximal anchor, 22 can be formed at the distal end 14 and proximal end 16, respectively. Each anchor 21, 22 is biased to a coiled configuration. Unlike a conventional stent which consists of only a single anchor located at a distal end, the stent 10 of this embodiment comprises an additional proximal anchor 22 adapted to be disposed in the bladder.

The body 12 of the stent 10 has a unique elongated tubular wall 23 formed by a plurality of filaments 25 which defined interstices 27 in the wall 23. With this configuration, the stent 10 can be stretched or elongated to a low-profile configuration, and compressed or compacted to a high-profile configuration.

Figure 2 illustrates an elongate member, or positioner, 30 which preferably has a tubular shaft terminating at a radiopaque marker, or distal tip 32. The positioner 30 is adapted to be threaded over a guidewire as illustrated in Figure 3. In particular, the positioner 30 includes a lumen 31 configured to receive a guidewire.

In operation, at least a portion of the distal section 17 is threaded onto the guidewire 34. This portion of the distal section 17 is hereinafter referred to as a threaded portion 36. The positioner 30 is then loaded onto the guidewire 34 proximally of the threaded portion 36 of the stent 10. All portions of the stent 10 which are not threaded onto the guidewire 34 are disposed outwardly of the guidewire 34 and the positioner 30, as the threaded portion 36 of the stent 10 is pushed by the positioner 30 to its operative site.

In the method of Figure 3, the threaded portion 36 of the stent 10 includes the marker 18 which has a tubular configuration and functions as a guide bushing. In this method, the guidewire 34 is threaded through the interstices 27 of the body portion 20 and through the marker 18 as illustrated in Figure 3.

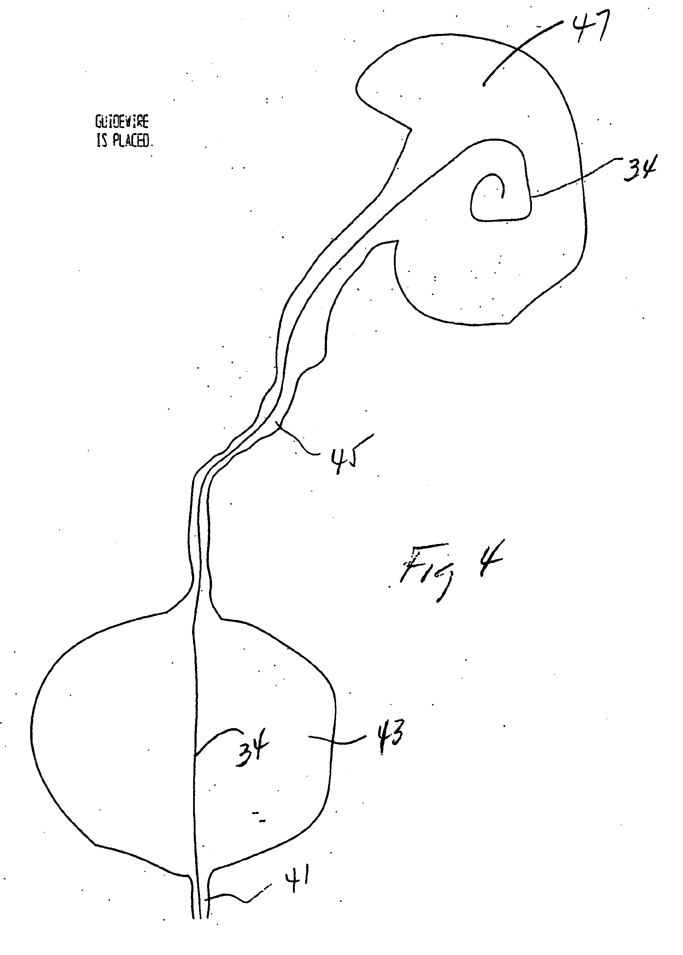

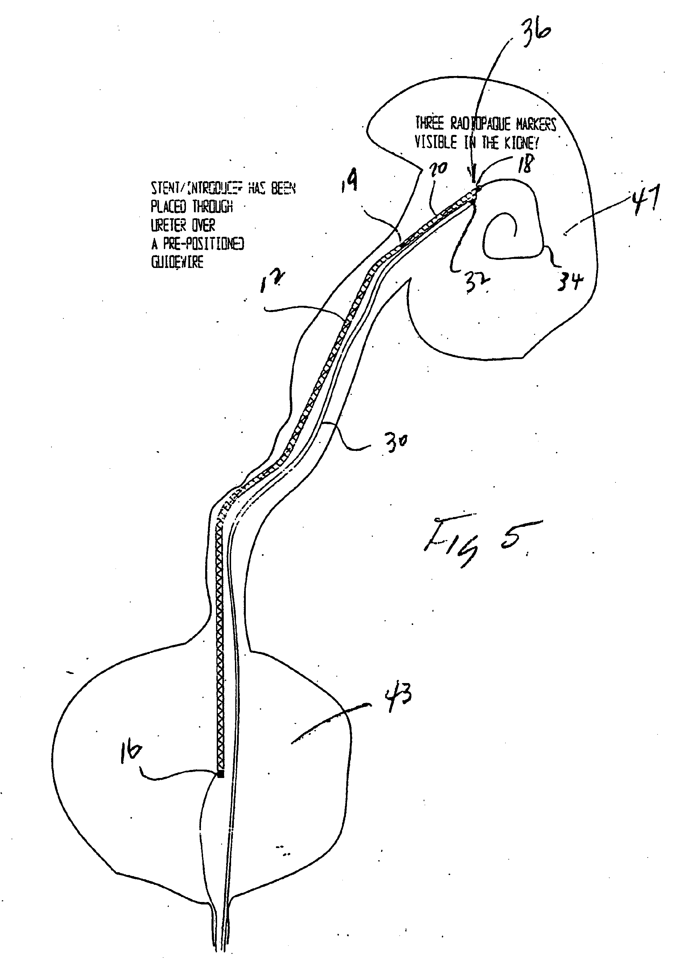

In Figure 4, a preferred process begins with placement of the guidewire 34 through the urethra 41, the bladder 43, the ureter 45, and into the kidney 47. As illustrated in Figures 3-5, the portion 36 of the distal section 17 is then threaded onto the guidewire 34 along with the positioner 30. As the positioner 30 is advanced through the bladder 43 and ureter 45, the threaded portion 36 is pushed along the guidewire 34 and into the kidney 47. In this manner, the stent 10 can be operatively positioned with its distal end 14 in the kidney 47, and its proximal end 16 in the bladder 43, as illustrated is Figure 5.

It will be noted that with this apparatus and method, the distal section 17 of the stent 10 is maintained in a straight, generally parallel relationship with the guidewire 34 and positioner 30. All portions of the stent body 12 which are proximal of the threaded portion 36 are stretched or elongated thereby providing the stent 10 with a low-profile configuration which is particularly advantageous during insertion.

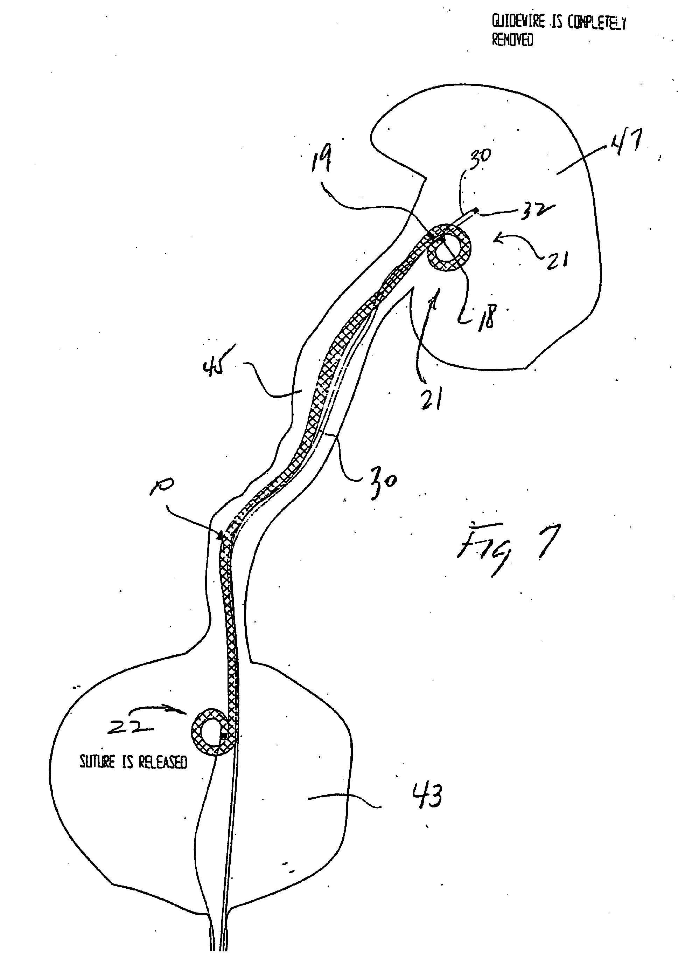

In order to deploy the stent 10, the guidewire 34 can be withdrawn proximally into the positioner 30. This will also withdraw the guidewire 34 from the threaded portion 36 of the stent 10 thereby enabling the distal anchor 21 to automatically assume its coil configuration, as illustrated in Figure 7. After the stent 10 has been deployed in its operative position, the guidewire 34 and positioner 30 can be withdrawn. Any tendency of the stent 10 to follow the positioner 30 will be opposed by the distal anchor 21 in the kidney 47.

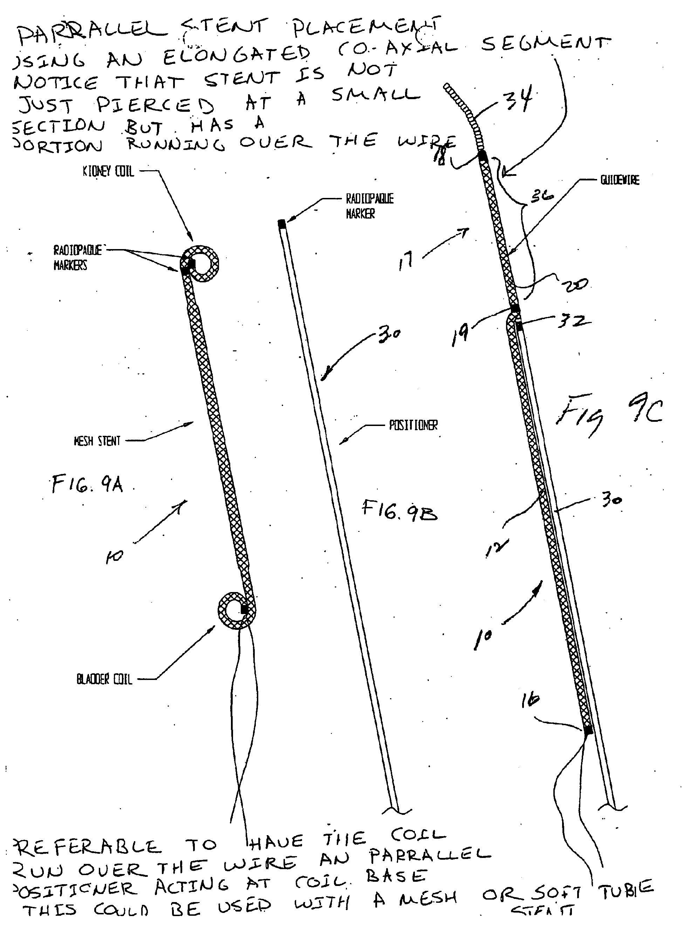

In the method and apparatus of Figures 9A-9C, the stent 10 and positioner 30 can be similar to those described and illustrated in Figures 1 and 2, respectively. In this case, Figure 9C differs from Figure 3 in that the threaded portion 36 includes not only the marker 18, but also the marker 19 and the body portion 20 extending therebetween. Thus, the entire distal section 17 can be threaded onto the guidewire and pushed distally along the guidewire 34 by the positioner 30.

It will be noted that in this method, the distal section 17 is also maintained in a generally parallel relationship with the guidewire 34. All portions of the stent body 12 proximal of the threaded portion 36 (proximal of the marker 19 in this case) extend outside the guidewire 34 and pusher 30. These portions tend to elongate upon insertion providing the stent 10 with the low-profile configuration.

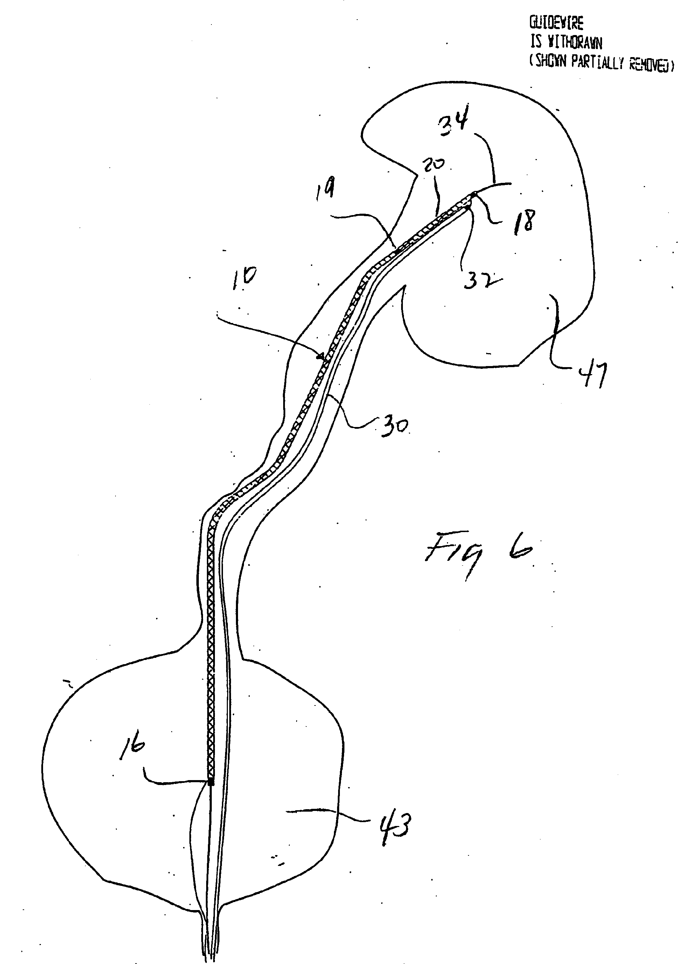

Deployment of the stent 10 in this case is similar to that previously discussed with reference to Figure 6. In this case, however, formation of the coiled distal anchor 21 begins to occur as soon as the guidewire 34 is withdrawn proximally of the marker 18. Complete formation of the distal anchor 21 does not occur until the guidewire 34 is withdrawn proximally from the body portion 20. Full deployment of the stent 10 does not occur until the guidewire 34 is fully withdrawn into the positioner 30.

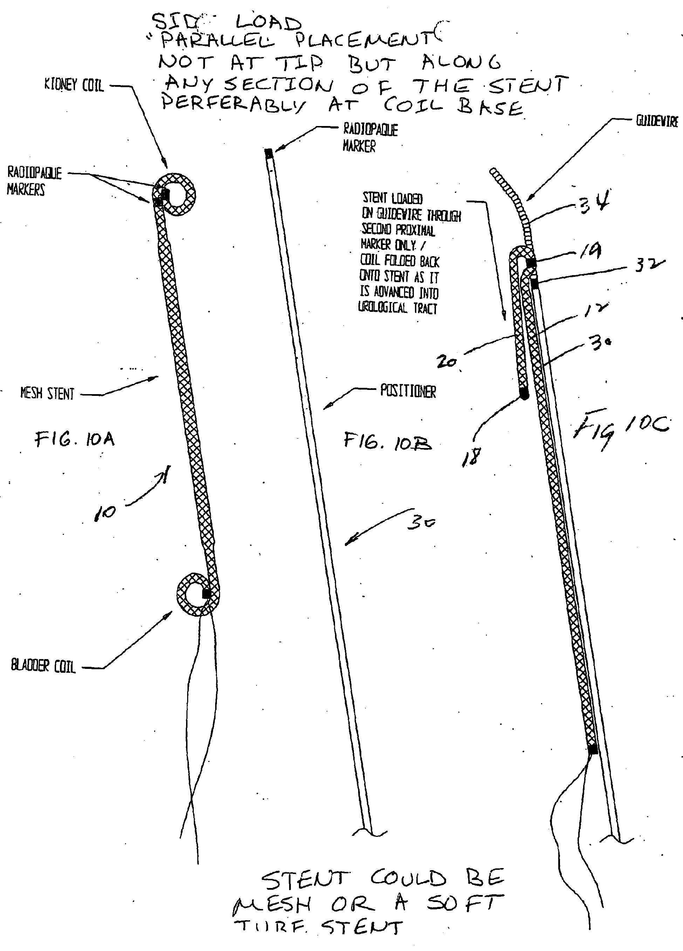

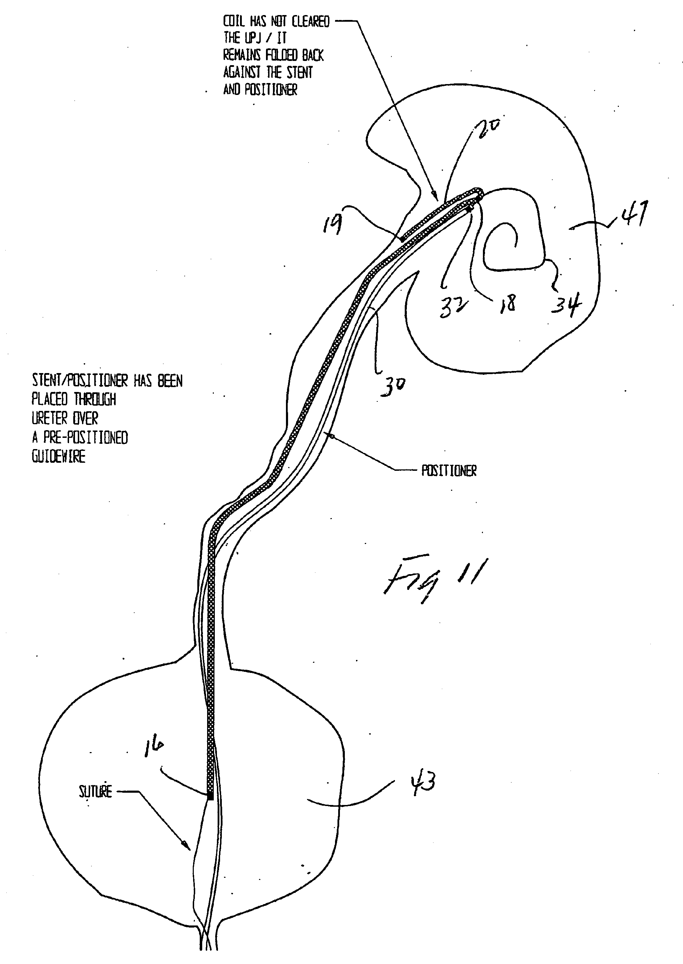

A further method is illustrated in Figures 10A-10C wherein the stent 10 and positioner 30 have the same configurations as previously discussed with reference to Figures 1 and 2. In this case, however, the threaded portion 36 of the stent 10 includes only the marker 19 which has a tubular configuration and functions as a guide bushing. The marker 18 and the body portions 20 are not threaded onto the guidewire 34, but rather are bent back on themselves into a parallel orientation with the remainder of the stent body 12, the guidewire 34, and the pusher 30. This orientation is illustrated in Figure 10C. Even here it will be noted that all portions of the stent 10, other than the threaded portion 36, are elongated into a low-profile state during insertion. In this case it will also be noted that only the threaded portion 36, the marker 19 in this case, is pushed by the pusher 30. The remaining portions of the stent 10 including the body portion 20 are pulled by the marker 19 into the operative position illustrated in Figure 11.

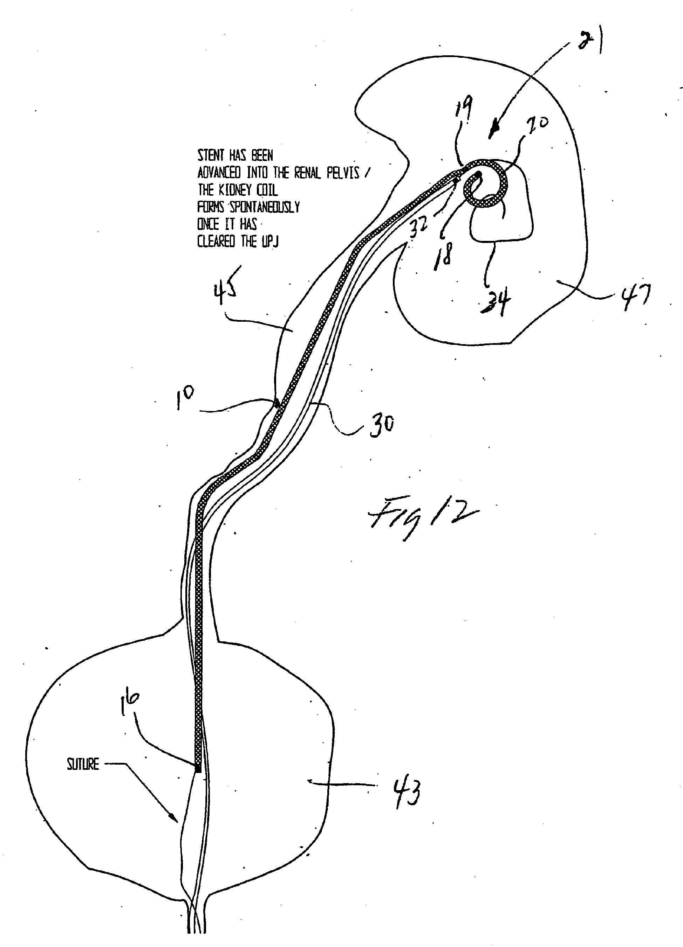

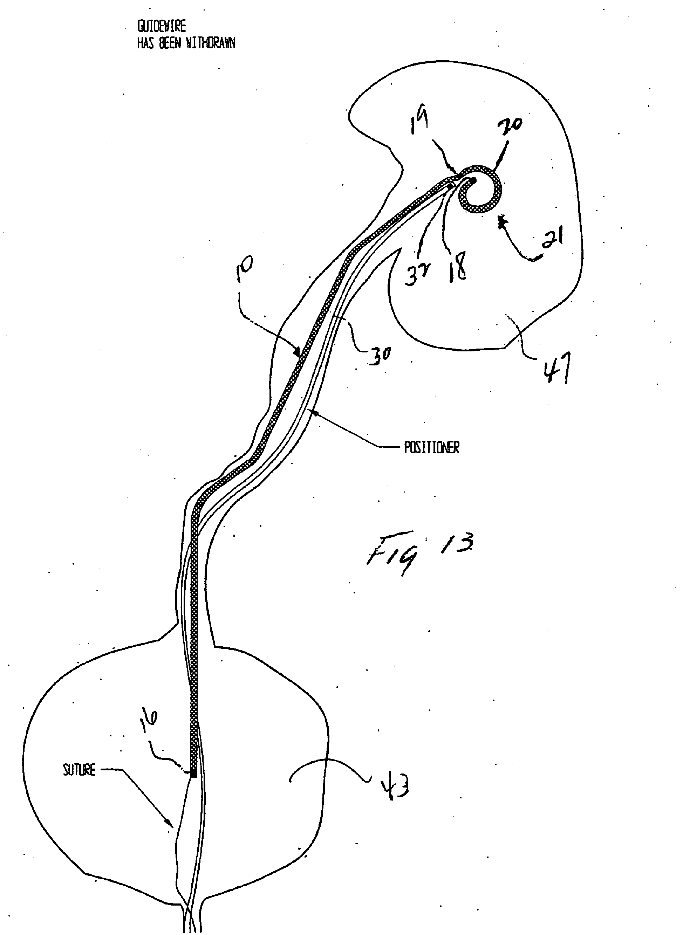

In this case, the distal anchor 21 will form completely as soon as the distal marker 18 clears the ureter 45. No withdrawal of the guidewire 34 is required for the complete formation of the distal anchor 21 as illustrated in Figure 12. Full deployment of the stent 10 is accomplished by withdrawing the guidewire 34 into the positioner 30 thereby releasing the threaded portion 36, in this case the marker 19. The positioner 30 and guidewire 34 can then be withdrawn leaving the stent operatively disposed as illustrated in Figure 13.

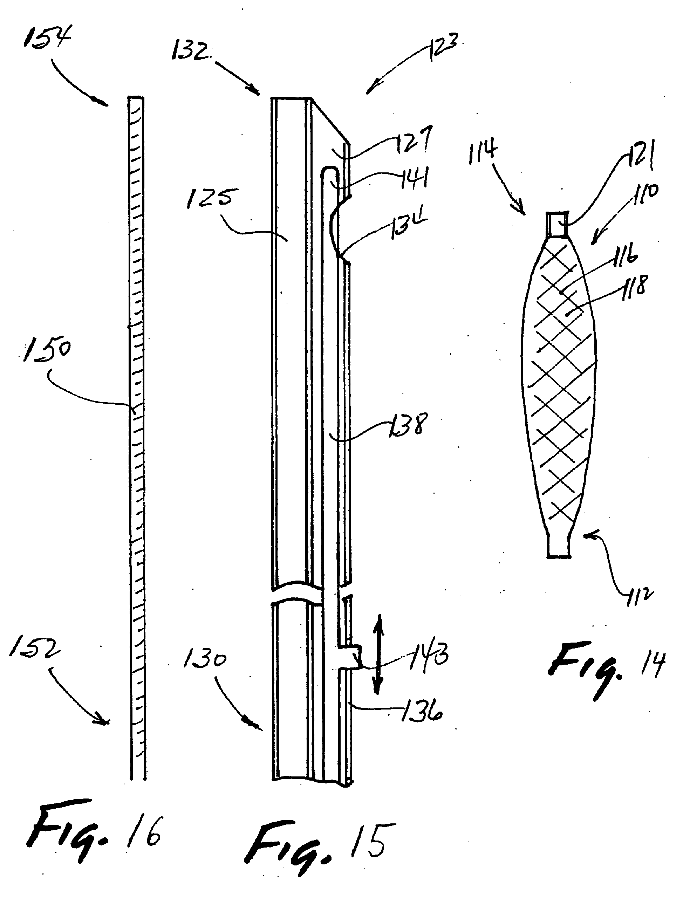

A second preferred embodiment of a stent is illustrated in Figure 14 and designated by the reference numeral 110, which includes a proximal end 112 and distal end 114. A plurality of filaments 116 extend between the ends 112 and 114, and are woven in a preferred embodiment so that they define interstices 118. Where the filaments 116 intersect, they can either be fixed or free to move relative to each other. Freedom of movement facilitates expansion of the stent 110 to a high profile, reduced length configuration, and contraction of the stent 110 to a low profile, increased length configuration.

At the distal end 114, the stent 110 is provided with a nose piece 121 which has a cylindrical configuration in a preferred embodiment. This nose piece 121 is of particular interest to the present invention.

In Figures 15-17, a delivery system includes an elongate tube 123 having at least two lumens 125 and 127 which extend generally between a proximal end 130 and a distal end 132. The lumen 125 is a thru-lumen open at both ends 130 and 132. The thru-lumen 125 is adapted to accommodate a conventional guidewire of the type illustrated in Figure 16 and designated by the reference numerical 150.

The lumen 127 may also be opened at both ends, but is preferably closed at the distal end 132. A scythe or side port 134 is formed near the distal end 132 while a preferred embodiment includes an axial slot 136 at the proximal end 130. An elongate rod 138 is disposed within the lumen 127 and provided with a distal end 141 which is moveable within the lumen 127. More specifically, the rod 138 can be provided with a thumb tab 143 which extends through the slot 136 and is moveable axially by the user. Operation of the thumb tab 143 moves the distal end 141 of the rod 138 from a position proximal of the scythe 134, across the scythe 134, to a position distal of the scythe 134.

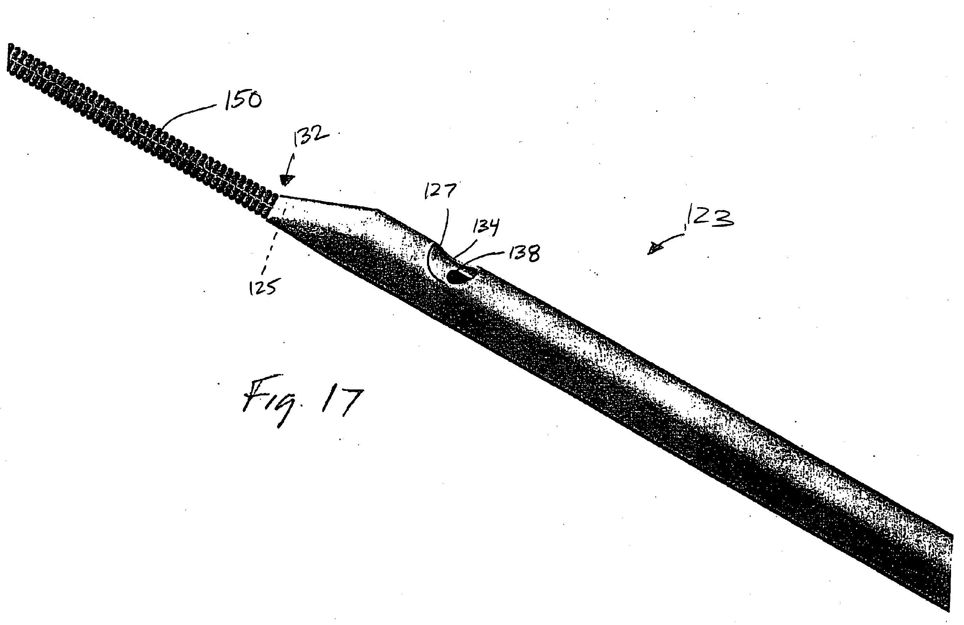

In operation, the stent 110 is releasably connected to the tube 123. In a preferred apparatus and method, the connection is accomplished by initially placing the rod 138 in its proximal position by operation of the finger tab 143. The nose piece 121 of the stent 110 is then positioned through the scythe 134 into the lumen 127 as illustrated in Figure 17. The rod 138 can then be advanced distally by operation of the finger tab 143. As the distal tip 141 moves across the scythe 134, it passes through the interstices 118 of the mesh and into the nose piece 121 of the stent 110. Further movement of rod 138 in the distal direction will lock the nose piece 121 into the lumen 127 while the remaining portions of the stent 110 are disposed outwardly of the tube 123.

The releasable attachment of the stent 110 to the tube 123 can be accomplished during the manufacturing process, in which case the combination, or system, 100 is provided to the user with the stent 110 already attached to the tube 122. Alternatively, the step of attaching the stent 110 to the tube 123 can occur during the operative procedure. In either event, the operative procedure begins with the placement of the guidewire 150 having a proximal end 152 and a distal end 154. This guidewire placement is accomplished in a manner well known in the art.

At this point in the process, the tube 123 can be threaded onto the guidewire 150. This is accomplished, as illustrated in Figure 17, by introducing the proximal end 152 of the guidewire 150 into the distal end 132 of the open lumen 125. The stent 110 may or may not be attached to the tube 123 during this initial guidewire insertion step.

If the stent 110 has not been attached to the tube 123, this attachment can now be undertaken in the manner previously discussed. Once again, the nose piece 121 of the stent 110 is inserted into the scythe 134 and the rod 138 advanced with the finger tab 143 until its distal end 141 extends through the interstices 118 and the nose piece 121 to releasably attach the stent 110 to the tube 123. The resulting configuration is illustrated in Figure 5. At this point, the tube 123 can be advanced along the guidewire 150 until the stent 110 is brought to its operative position in the body conduit, such as the ureter.

Once the stent 110 is operatively disposed, it can be released from the tube 123 to permit withdrawal of the tube 123 and the guidewire 150. Release of the stent 110 is accomplished in a preferred method by reversing the attachment steps. The rod 138 is initially moved proximally by operation of the finger tab 143 until it clears the nose piece 121 and filaments 116. At this point, the stent 110 is released from the tube 123 to permit removable of the tube 123 and the guidewire 150. The stent 110 is left in its operative position within the body conduit such as the ureter.

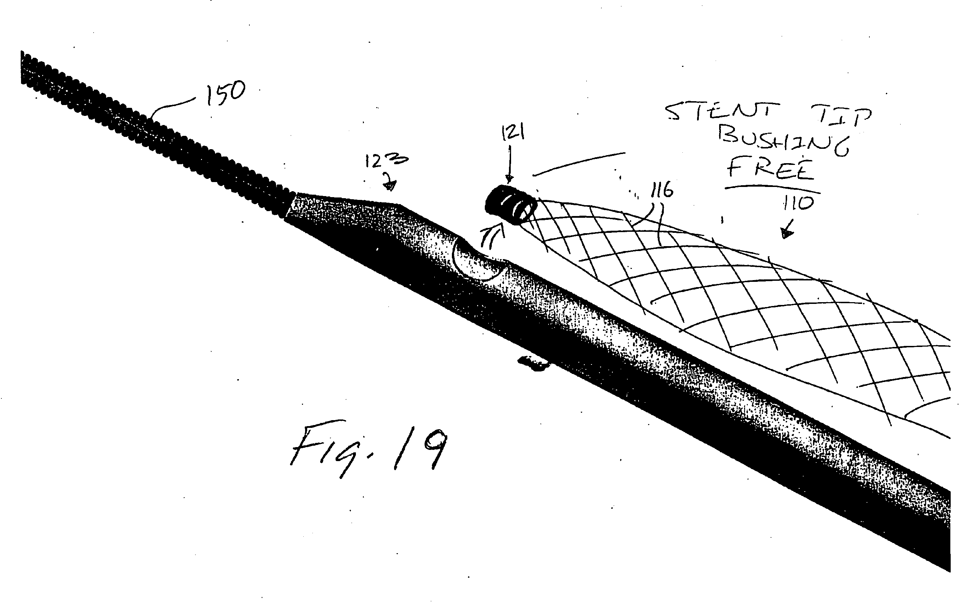

The step of releasing the stent 110 from the tube 123 is illustrated in Figure 19. In a preferred embodiment, the stent 110 is provided with memory characteristics so that the distal end 114 and proximal end 112, shown in Figure 14, automatically achieve a coiled configuration to anchor the stent 110 in the kidney and bladder, respectively, upon release from the tube 123.

In the second preferred embodiment of the stent deployment system 100, it will be appreciated that the guidewire 150 may be moved independently from the stent 110. In particular, the guidewire 150 need not be retracted in order to deploy the stent 110. Therefore, the system 100 may be preferable in situations where the guidewire 150 is desired to be continually manipulated while the stent 110 is independently deployed.

Many alterations and modifications may be made by those having ordinary skill in the art without departing from the scope of the invention. Therefore, it must be understood that the illustrated embodiments have been set forth only for the purposes of examples and that they should not be taken as limiting the invention as defined by the following claims.

## Claims

1. A surgical combination for stenting a ureter, comprising:
a guidewire (34) having a distal end, the guidewire being adapted to be positioned in the ureter;
a stent (10) having a body (12), a distal anchor (21), and a radiopaque marker (18, 19) disposed around the body proximally of the distal anchor; **characterized in that**
the marker is sized and configured for distal movement along the guidewire to an operative site; and also comprising
a positioner (30) having a tubular configuration and being adapted for distal movement along the guidewire to hold the marker at the operative site when the guidewire is withdrawn from the ureter.

2. A surgical combination as claimed in Claim 1, wherein:
the distal anchor has a straight configuration and a curled configuration;
the distal anchor is able to be disposed in the straight configuration when the marker is moved distally along the guidewire; and
the distal anchor is able to be disposed in the curled configuration when the guidewire is withdrawn from the operative site.

3. The surgical combination as claimed in Claim 2, wherein the distal anchor of the stent is biased to the curled configuration.

4. A surgical combination as claimed in any preceding claim, the stent further comprising a proximal anchor (22), wherein
the distal anchor comprises a first pigtail coil; and
the proximal anchor comprises a second pigtail coil.

5. A surgical combination as claimed in any preceding claim, wherein the marker comprises a guide bushing.

6. A surgical combination as claimed in Claim 5, wherein
a distal end (14) of the stent body (12) has properties for being bent back on itself in a straight configuration when the guide bushing is moved along the guidewire (34) through a body conduit.

7. A surgical combination as claimed in any preceding claim,
wherein the positioner includes a first lumen (124) and a second lumen (127);
the first lumen being sized and configured to receive the guidewire; and
the second lumen being adapted to receive at least a portion of the stent in a releasably locked relationship.

8. A surgical combination as claimed in Claim 7, wherein:
a scythe (134) is formed in the positioner extending into the second lumen; and
a rod (138) is disposed in the second lumen and is movable between a first locking position wherein the rod engages the portion of the stent within the second lumen and a second releasing position wherein the rod disengages the portion of the second stent in the second lumen.

9. A surgical combination as claimed in Claim 8, wherein:
the portion of the stent comprises a distal end (114) of the stent.

## Patentansprüche

1. Eine chirurgische Kombination zum Einsetzen einer Schiene in einen Harnleiter, wobei die Kombination umfasst:
einen Führungsdraht (34) mit einem distalen Ende, wobei der Führungsdraht zur Positionierung in dem Harnleiter angepasst ist;
eine Schiene (10) mit einem Körper (12), einem distalen Anker (21) und einem röntgendichten Marker (18, 19), der um den Körper herum proximal zum distalen Anker angeordnet ist; **dadurch gekennzeichnet, dass**
der Marker zur distalen Bewegung entlang des Führungsdrahts zu einem Operationsort dimensioniert und konfiguriert ist; und wobei sie ebenfalls umfasst:
eine Positionierungsvorrichtung (30) mit einer röhrenförmigen Konfiguration, die zur distalen Bewegung entlang des Führungsdrahts angepasst ist und dazu dient, den Marker am Operationsort zu halten, wenn der Führungsdraht aus dem Harnleiter zurückgezogen wird.

2. Eine chirurgische Kombination gemäß Anspruch 1, wobei:
der distale Anker eine gerade Konfiguration und eine gekrümmte Konfiguration aufweist;
der distale Anker in der geraden Konfiguration angeordnet werden kann, wenn der Marker distal entlang des Führungsdrahts bewegt wird; und
der distale Anker in der gekrümmten Konfiguration angeordnet werden kann, wenn der Führungsdraht aus dem Operationsort zurückgezogen wird.

3. Eine chirurgische Kombination gemäß Anspruch 2, wobei der distale Anker der Schiene für die gekrümmte Konfiguration vorbelastet ist.

4. Eine chirurgische Kombination gemäß einem beliebigen der vorstehenden Ansprüche, wobei die Schiene weiterhin einen proximalen Anker (22) umfasst, wobei
der distale Anker eine erste Pigtail-Krümmung umfasst; und
der proximale Anker eine zweite Pigtail-Krümmung umfasst.

5. Eine chirurgische Kombination gemäß einem beliebigen der vorstehenden Ansprüche, wobei der Marker eine Führungsbuchse umfasst.

6. Eine chirurgische Kombination gemäß Anspruch 5, wobei
ein distales Ende (14) des Schienenkörpers (12) Eigenschaften aufweist, die es ermöglichen, in einer geraden Konfiguration in sich selbst zurückgebogen zu werden, wenn die Führungsbuchse entlang des Führungsdrahts (34) durch einen Körperleiter bewegt wird.

7. Eine chirurgische Kombination gemäß einem beliebigen der vorstehenden Ansprüche,
wobei die Positionierungsvorrichtung ein erstes Lumen (124) und ein zweites Lumen (127) umfasst;
wobei das erste Lumen zur Aufnahme des Führungsdrahts dimensioniert und konfiguriert ist; und
das zweite Lumen zur Aufnahme von zumindest einem Abschnitt der Schiene in einer lösbar eingerasteten Beziehung angepasst ist.

8. Eine chirurgische Kombination gemäß Anspruch 7, wobei:
eine Sense (134) in der Positionierungsvorrichtung ausgebildet ist, die sich in das zweite Lumen hinein erstreckt; und
ein Stab (138) in dem zweiten Lumen angeordnet ist, der zwischen einer ersten Einrastposition, in welcher der Stab Eingriff mit dem Abschnitt der Schiene im zweiten Lumen nimmt, und einer zweiten Löseposition, in welcher der Stab den Eingriff mit dem Abschnitt der zweiten Schiene im zweiten Lumen löst, beweglich ist.

9. Eine chirurgische Kombination gemäß Anspruch 8, wobei:
der Abschnitt der Schiene ein distales Ende (114) der Schiene umfasst.

## Revendications

1. Une combinaison chirurgicale de pose de stent dans un urètre, comprenant :
un fil guide (34) ayant une extrémité distale, le fil guide étant adapté pour être positionné dans l'urètre ;
un stent (10) ayant un corps (12), une ancre distale (21) et un marqueur radio-opaque (18, 19) disposé autour du corps proximalement de l'ancre distale ; **caractérisé en ce que**
le marqueur est dimensionné et configuré pour un déplacement distal le long du fil guide jusqu'à un site opératoire ; et comprenant également
un positionneur (30) ayant une configuration tubulaire et étant adapté pour un déplacement distal le long du fil guide afin de maintenir le marqueur sur le site opératoire lorsque le fil guide est retiré de l'urètre.

2. Une combinaison chirurgicale selon le procédé de la Revendication 1,
l'ancre distale ayant une configuration rectiligne et une configuration en spirale ;
l'ancre distale pouvant être disposée en la configuration rectiligne lorsque le marqueur est déplacé distalement le long du fil guide ; et
l'ancre distale pouvant être disposée en la configuration en spirale lorsque le fil guide est retiré du site opératoire.

3. Une combinaison chirurgicale selon le procédé de la Revendication 2, l'ancre distale du stent étant sollicitée par la configuration en spirale.

4. Une combinaison chirurgicale selon une quelconque revendication précédente, le stent comportant en outre une ancre proximale (22), dans laquelle
l'ancre distale comprend une première queue recourbée ; et
l'ancre proximale comprend une deuxième queue recourbée.

5. Une combinaison chirurgicale selon une quelconque revendication précédente, le marqueur comprenant une douille de guidage.

6. Une combinaison chirurgicale selon le procédé de la Revendication 5, dans laquelle
une extrémité distale (14) du corps du stent (12) a des propriétés de recourbement sur elle-même en une configuration rectiligne lorsque la douille est déplacée le long du fil guide (34) à travers un conduit corporel.

7. Une combinaison chirurgicale selon une quelconque revendication précédente, dans laquelle
le positionneur comporte une première lumière (124) et une deuxième lumière (127) ;
la première lumière est dimensionnée et configurée de manière à recevoir le fil guide ; et
la deuxième lumière est adaptée de manière à recevoir au moins une partie du stent en une relation verrouillée à libération.

8. Une combinaison chirurgicale selon le procédé de la revendication 7, dans laquelle
une faux (134) est formée dans le positionneur s'étendant dans la deuxième lumière et une tige (138) est disposée dans la deuxième lumière et est mobile entre une première position à verrouillage dans laquelle la tige s'engage dans la partie du stent se trouvant dans la deuxième lumière ; et une deuxième position à libération dans laquelle la tige se désengage de la partie du deuxième stent dans la deuxième lumière.

9. Une combinaison chirurgicale selon le procédé de la revendication 8 dans laquelle:
la portion du stent comprend une extrémité (114) distale du stent.
